# EUROPEAN PATENT SPECIFICATION

(11) **EP 2 500 013 B1**
(45) Date of publication and mention of the grant of the patent: **02.10.2019**
(21) Application number: 12001737.1
(22) Date of filing: 14.03.2012
(51) Int. Cl.: A61K 9/16, A61K 9/20, A61K 31/4725

(54) **Pharmaceutical composition comprising solifenacin**
Pharmazeutische Zusammensetzung mit Solifenacin
Composition pharmaceutique comprenant de la solifénacine

(30) Priority: 15.03.2011 IN DE07282011
(43) Date of publication of application: 19.09.2012
(73) Proprietor: Alfred E. Tiefenbacher (GmbH & Co. KG), 22767 Hamburg (DE)
(72) Inventor: Manohar Lal, Pasahn, Thane (West) - 400 602 Maharashtra (IN); Rallabandi Ramesha, Chary, Kukatpally, Hyderabad - 500 072 Andhra Pradesh (IN); Abhay Ramakant, Joshi, Chandgad Kolhapur (Dist) Maharashtra (IN); Rillmann, Thomas, 76756 Bellheim (DE)
(74) Representative: Hamm&Wittkopp Patentanwälte PartmbB

(56) References cited:
- EP-A1- 1 728 791
- EP-A1- 1 911 444
- WO-A2-2008/013851
- WO-A2-2009/135947
- WO-A2-2009/135950
- WO-A2-2010/012459
- WO-A2-2010/097243
- US-A1- 2010 136 110
- US-A1- 2010 233 260
- DATABASE WPI Section Ch, Week 200450 Thomson Scientific, London, GB; Class A96, AN 2004-520611 XP002681198, SUGIMOTO M; SUGIMOTO M, JP: "Orally disintegrable formulation for treating dysuria accompanying renal hypertension, prostatic hypertrophy and pheochromocytoma, contains drug having dysuria treating effect as active ingredient", -& JP 2004 175796 A ((ASAH) ASAHI KASEI PHARMA KK) 24 June 2004 (2004-06-24)

## Description

The present invention relates to a pharmaceutical composition in the form of a solid oral dosage form comprising solifenacin or a pharmaceutically acceptable salt thereof, preferably crystalline solifenacin succinate.

Solifenacin is a competitive muscarinic acetylcholine receptor antagonist belonging to the class of urinary antispasmodic, Solifenacin is marketed under the tradename Vesikur® or Vesicare® as a film-coated tablet that contains either 5 mg or 10 mg solifenacin succinate. The tablet is approved for the symptomatic treatment of urge incontinence and/or increased urinary frequency and urgency as may occur in patients with overactive bladder syndrome.

It is commonly known that solifenacin may undergo chemical degradation as oxidative decomposition, photo degradation, hydrolysis etc., so that the drug may contain a substantial amount of impurities. In order to reduce the formation of impurities in the drug over time various pharmaceutical compositions have been suggested, in which solifenacin is stabilized.

According to EP-A-1 728 791 especially the amorphous form of solifenacin succinate is chemically unstable. It was found that if solifenacin succinate is subjected to a fluidized bed granulation process and subsequently converted into tablets, the amount of a main degradation product (designated as "F1") increased over time when the tablet was subjected to usual stability tests, In order to improve the stability of solifenacin within the tablet it was suggested to use the crystalline form of solifenacin or a pharmaceutically acceptable salt thereof, in which form the amorphous content of the drug is adjusted to a range showing no influence on product stability. According to EP-A-1 728 791 solifenacin succinate is unstable, if the amorphous content in the drug exceeds 77 %.

The patent application also discloses that wet granulation is the preferred technique for preparing stable solifenacin containing pharmaceutical compositions. The conditions employed in the wet granulation process must be carefully adjusted in order to minimize the generation of amorphous solifenacin. In this regard, the application suggests reducing either the spray rate of the binder solution during granulation or the total amount of the binder solution. It was also found that polyethylene glycol (PEG), e.g. Macrogol 6000, is capable of suppressing the generation of amorphous solifenacin succinate to a larger extent than hydroxypropylmethyl cellulose (HPMC), e.g. HPMC 2910, or maize starch, if used as a binder.

EP-A-1 832 288 discloses a stable particulate pharmaceutical composition comprising solifenacin, preferably solifenacin succinate, wherein the drug is stabilized by the use of a binder having a glass transition point or melting point lower than 174°C. Preferred examples of the binder include polyethylene glycol, polyoxyethylene/ polyoxypropylene block copolymer and hydroxypropyl cellulose (HPC). The particulate pharmaceutical composition is prepared by coating a core particle, which is preferably made from crystalline cellulose (Celphere), with a solution containing solifenacin and the binder. The coated core particle may subsequently be compressed into orally disintegrating tablets.

According to WO 2008/128028 even solifenacin succinate in amorphous or substantially amorphous form may be prepared into stable solid pharmaceutical compositions, It is reported that solifenacin undergoes oxidative decomposition. In order to improve the stability of solifenacin, antioxidants should be contained in the pharmaceutical composition. Preferred antioxidants reported in WO 2008/128028 are butylated hydroxyanisole, butylated hydroxytoluene, ascorbic acid or a salt thereof, sulfites, such as sodium hydrogen sulfite, a salt of edetic acid, such as sodium EDTA, propyl gallate, tocopherol, and the like. The application describes capsules and tablets which are preferably made from premix compositions containing solifenacin succinate. The premix compositions are preferably prepared by subjecting a solution containing the drug, the antioxidant and a binder to wet granulation,

WO 2009/012987 suggests the stabilization of amorphous solifenacin by covering or surrounding the drug with a stabilizer. In a preferred embodiment stable amorphous solifenacin is prepared by spray drying a solution containing solifenacin succinate and a stabilizer. Examples of the stabilizer include PEG, HPMC, methyl cellulose (MC), polyvinylpyrrolidone, and the like. Alternatively, stabilized amorphous solifenacin is obtained by melt extrusion, wherein the stabilizer may be selected from vinylpynolidone/vinyl acetate copolymer (copovidone), polyvinyl acetate, polymethacrylate, mannitol, and the like.

According to US 2010/0273825 solifenacin decomposes time-dependently by the influence of oxidation. It was commonly known that this decomposition is inhibited in the presence of a stabilizer. However, the stabilizers suggested in the state of the art did not suppress discoloration or coloration of the final pharmaceutical composition containing solifenacin. It was found that the discoloration or coloration of a solid pharmaceutical composition containing amorphous solifenacin is avoided, if the composition contains a stabilizer selected from citric acid or a salt thereof, sodium pyrosulfite and a salt of edetic acid.

As an alternative approach for overcoming the stability problems encountered with solifenacin, WO 2010/097243 suggests the preparation of a solid pharmaceutical composition containing solifenacin in the absence of solvents, in particular in the absence of water. As a manufacturing method, which does not require the presence of solvents, direct compression and dry granulation processes are mentioned. The pharmaceutical compositions disclosed in WO 2010/097243 contain solifenacin or a pharmaceutical salt thereof in crystalline form.

EP-A1-1 911 444 discloses the preparation of quickly disintegrating tablets from a mixture comprising (a) solifenacin succinate-containing coated microparticles and (b) granulated particles comprising the excipients mannitol and maltose. In this document, polyethylene glycol is used to prevent the drug from becoming amorphous and unstable.

In view of the state of the art described above, it was an object of the present invention to provide an alternative approach for the preparation of a pharmaceutical composition in the form of a solid oral dosage form, in which solifenacin or a pharmaceutically acceptable salt thereof is chemically stable.

This object was solved by the subject matter as defined in the claims.

It was surprisingly found that the stability of solifenacin can be substantially improved, if the drug is not exposed to moisture during the preparation of the solid pharmaceutical composition. As discussed in EP-A-1 728 791 or WO 2010/097243 large quantities of water should be avoided in the preparation of solid pharmaceutical compositions containing solifenacin. Preferably, the use of water should be avoided at all, because water may promote the conversion of crystalline solifenacin into its amorphous state. However, amorphous solifenacin is particularly susceptible to chemical degradation.

Due to the fact that the chemical degradation of solifenacin is promoted by the presence of water, the pharmaceutical excipients contained in the solid pharmaceutical composition together with the drug have to be carefully selected. Even if the solid pharmaceutical composition is prepared in the absence of solvents, i.e. using dry techniques as direct compression and dry granulation only, degradation of the drug may still occur, if the drug is mixed with pharmaceutical excipients containing a certain amount of water. In order to lower the risk that water contained in such pharmaceutical excipients adversely affects the stability of solifenacin or a pharmaceutically acceptable salt thereof, the preparation of a pharmaceutical composition, which method would provide the possibility to dry or adjust the water content of the pharmaceutical excipients, while the drug's stability is not adversely affected, would be useful.

This is achieved with the pharmaceutical composition in the form of a solid oral dosage form according to claim 1, which comprises solifenacin or a pharmaceutically acceptable salt thereof as a pharmaceutical active ingredient and excipient granules prepared from at least two pharmaceutical excipients. Contrary to the commonly known dry granulation techniques, the granules contained in the pharmaceutical composition according to the present invention do not contain solifenacin, so that even comparatively wet pharmaceutical excipients as well as wet granulation techniques may be employed in the preparation of the pharmaceutical composition, because the wet granules may be dried to such an extent that their water content does not cause any stability problems for the drug.

In a preferred embodiment of the present invention, the pharmaceutical composition contains a crystalline solifenacin salt, preferably crystalline solifenacin succinate. The pharmaceutical composition according to the present invention, e.g. a tablet or film-coated tablet, contains amongst the pharmaceutically active ingredient pharmaceutical excipients as filler, binder, disintegrant, antioxidant, glidant and lubricant. These pharmaceutical excipients may be contained in the excipient granules or included as extragranular components. Preferably, the excipient granules contain at least one filler, at least one binder and optionally one disintegrant, while lubricants and optionally glidants are contained as extragranular components only.

Examples of the filler contained in the pharmaceutical composition and, in particular, in the excipient granules are cellulose, e.g. microcrystalline cellulose and powdered cellulose; sugars, e.g. glucose, lactose (anhydrous or monohydrate), maltose, and sucrose; sugar alcohols, e.g. isomalt, lactitol, maltitol, mannitol, sorbitol, and xylitol; starch (maize, potato, wheat); a calcium salt, e.g. calcium hydrogen phosphate; and the like.

Examples of the binder contained in the excipient granules include methyl cellulose (MC), hydroxypropylmethyl cellulose (hypromellose, HPMC), hydroxypropyl cellulose (hyprolose, HPC), low-substituted hydroxypropyl cellulose (L-HPC), carboxymethylcellulose sodium (CMC sodium), polyethylene glycol (PEG), maltodextrin, pregelatinized starch (Starch 1500), polyvinylpyrrolidone (povidone, PVP), vinylpyrrolidone/vinyl acetate copolymer (copovidone), and polyvinyl alcohol/polyethylene glycol graft copolymer (Kollicoat® IR).

Examples of the disintegrant, which may be contained in the excipient granules and/or as an extragranular component, include croscarmellose sodium, sodium starch glycolate, polyvinylpolypyrrolidone (crospovidone), and the like.

Examples of the antioxidant, which may be contained in the excipient granules and/ or as an extragranular component, include α-tocopherol (vitamin E), ascorbic acid, ascorbyl palmitate, butylated hydroxyanisole (BHA), butylated hydroxytoluene (BHT), citric acid, edetic acid and salts thereof (preferably disodium EDTA), monothioglycerol, potassium metabisulfite, gallate as ethyl, propyl, octyl or dodecyl gallate (preferably propyl gallate), sodium bisulfite, sodium metabisulfite (sodium pyrosulfite), sodium sulfite and tartaric acid.

As glidants, silicon dioxide, talc and the like may be used, while magnesium stearate, calcium stearate, stearic acid, sodium stearyl fumarate, hydrogenated vegetable oil, glycerol dibehenate (Compritol®), and glycerol palmitostearate or glycerol distearate (Precirol®) are examples of suitable lubricants. Preferably a mixture of a glidant and lubricant is used, e.g. a mixture of silicon dioxide (colloidal anhydrous silica; Aerosil® 200) and a lubricant selected from magnesium stearate, glycerol dibehenate and glycerol distearate.

In a preferred embodiment of the present invention the pharmaceutical composition is a tablet or tablet core consisting of the said excipient granules, the solifenacin drug, and, as an extragranular component, at least one pharmaceutical excipient selected from lubricants and glidants.

It has been found that the chemical stability of solifenacin is safeguarded if the pharmaceutical composition's water content is adjusted to 1 % to 10 %, preferably 2 % to 8 %, more preferred 3 % to 6 % and most preferred 4 % to 5 % as determined by Karl Fischer titration.

The pharmaceutical composition according to the present invention is prepared by a process comprising the method steps of:
i) preparing excipient granules from at least two pharmaceutical excipients by wet granulation,
ii) mixing said excipient granules with the pharmaceutically active ingredient and optionally with additional pharmaceutical excipients to obtain a mixture,
iii) subjecting the mixture obtained in method step (ii) to compression, and
iv) optionally milling the compacted mass obtained in method step (iii), optionally mixing the milled mass with pharmaceutical excipients, and subjecting the milled mass/mixture to compression.

In a preferred embodiment of the process according to the present invention, the wet granulation is conducted using a High Shear Mixer Granulator or a Fluid Bed Granulator.

In method step (iii) or (iv), a tablet containing solifenacin or a pharmaceutically acceptable salt thereof is obtained, which tablet may be film-coated with a solution containing ethylcellulose or hypromellose, e.g. Opadry®.

The following examples are intended to further illustrate the present invention.

### Examples

The measurement of the water content of the pharmaceutical composition using Karl Fischer titration was conducted as described in chapter 2.5.12 of the European Pharmacopoeia (EP) 6.8. Stability testing was conducted according to guideline Q 1 A (R2) of International Conference on Harmonization (ICH).

### Example 1

| Ingredients | Milligram/tablet | |
|---|---|---|
| **Stage - A (Dry Mix)** | | |
| Lactose Monohydrate (Granulac 200) | 121.00 | |
| Maize Starch | 15.00 | |

| **Stage - B (Granulation)** | | |
|---|---|---|
| Hypromellose (HPMC E5) | 3.00 | |
| Water, Purified | q.s. | |

| **Stage - C (Blending & Lubrication)** | | |
|---|---|---|
| Excipient Granules | 139.00 | |
| Solifenacin Succinate | 10.00 | |
| Magnesium Stearate | 1.00 | |
| ***Core Tablet weight*** | ***150.00*** | |

| **Stage - D (Coating)** | **Option A** | **Option B** |
|---|---|---|
| Hypromellose (Opadry® Yellow 13B82402) | 4.00 | - |
| Ethylcellulose (Ethocel 7 cP) | - | 0.40 |
| Hypromellose (HPMC E5) | - | 3.60 |
| Isopropyl Alcohol | - | q.s. |
| Water, Purified | q.s. | q.s. |
| ***Coated Tablet weight*** | ***154.00*** | ***154.00*** |

| | | |
|---|---|---|
| Opadry® Yellow 13B82402: further ingredients are titanium dioxide, polysorbate 80, yellow iron oxide and macrogol | | |

### Process:

A dry mix containing lactose monohydrate and maize starch was treated with a binder solution containing hypromellose dissolved in water and mixed in a High Shear Mixer Granulator. The wet mass was dried in a Fluid Bed drier and the obtained granules were milled. Thereafter, the granules were blended with solifenacin succinate and magnesium stearate to obtain a tablet mass which was compressed into tablets. Finally, the tablets were film-coated.

### Comparison Example 1 (wet granulation)

| Ingredients | Milligram/tablet |
|---|---|
| **Stage - A (Dry Mix)** | |
| Solifenacin Succinate | 10.07 |
| Lactose Monohydrate (Granulac 200) | 105.33 |
| Maize Starch | 30.00 |

| **Stage - B (Granulation)** | |
|---|---|
| Hypromellose (HPMC E5) | 4.00 |
| Water, Purified | q.s. |

| **Stage - C (Blending & Lubrication)** | |
|---|---|
| Magnesium Stearate | 0.60 |
| ***Core Tablet weight*** | ***150.00*** |

| **Stage - D (Coating)** | |
|---|---|
| Hypromellose (Opadry® Pink 03FS540018) | 4.00 |
| Water, Purified | q.s, |
| ***Coated Tablet weight*** | ***154.00*** |

| | |
|---|---|
| Opacity® Pink 03FS540018: further ingredients are titanium dioxide, talc, red iron oxide and macrogol | |

### Process:

A dry mix containing solifenacin succinate, lactose monohydrate and maize starch was treated with a binder solution containing hypromellose dissolved in water and mixed in a High Shear Mixer Granulator. The wet mass was dried in a Fluid Bed drier and the obtained granules were then milled. Thereafter, the granules were blended with magnesium stearate to obtain a tablet mass which was compressed into tablets. Finally, the tablets were film-coated.

### Comparison Example 2 (direct compression)

| Ingredients | Milligram/tablet |
|---|---|
| **Stage-A (Dry Mix)** | |
| Solifenacin Succinate | 10.05 |
| Lactose Anhydrous DCL (Supertab 21AN) | 100.95 |
| Maize Starch | 30.00 |
| Hypromellose (HPMC E5) | 7.50 |
| Magnesium Stearate | 1.50 |
| ***Core Tablet weight*** | ***150.00*** |

| **Stage** - **B (Coating)** | |
|---|---|
| Hypromellose (Opadry® Pink 03FS540018) | 4.00 |
| Water, Purified | q.s. |
| ***Coated Tablet weight*** | ***154.00*** |

| | |
|---|---|
| Opadry® Pink 03FS540018: further ingredients are titanium dioxide, talc, red iron oxide and macrogol | |

### Process:

A dry mix containing solifenacin succinate, lactose monohydrate, maize starch, hypromellose and magnesium stearate was compressed into tablets, and finally film-coated.

### Example 2

**Table. Stability test (40°C/75 % relative humidity)**

| | **Example 1** | **Comparison example 1** | **Comparison example 2** |
|---|---|---|---|
| Initial | Nil (Option A) | 0.06 | 0.01 |
| | Nil (Option B) | | |
| 2weeks | 0.02 (Option A) | - | 0.05 |
| | Nil (Option B) | | |
| 1 month | 0.05 (Option A) | 0.79 | 0.09 |
| | 0.01 (Option B) | | |
| 2 months | 0.07 (Option A) | - | 0.13 |
| | 0.01 (Option B) | | |
| 3 months | 0.07 (Option A) | - | 0.17 |
| | 0.02 (Option B) | | |
| 6 months | 0.12 (Option A) | - | 0.19 |
| | 0.10 (Option B) | | |

### Example 3

| Ingredients | Milligram/tablet |
|---|---|
| **Stage - A (Dry mix)** | |
| Lactose Monohydrate (Granulac 200) | 117.00 |
| Maize starch | 15.00 |

| **Stage - B (Granulation)** | |
|---|---|
| Hypromellose (HPMC E5) | 3.00 |
| Water, Purified | q.s. |

| **Stage - C (Blending & Compaction)** | |
|---|---|
| Excipient Granules | 135.00 |
| Solifenacin Succinate | 10.00 |
| Hypromellose (HPMC E5) | 3.00 |
| Magnesium Stearate | 0.50 |

| **Stage - D (Blending & Lubrication)** | |
|---|---|
| Stage C compacted blend | 148.50 |
| Magnesium Stearate | 1.500 |
| ***Core Tablet weight*** | ***150.00*** |

| **Stage** - **E (Coating)** | |
|---|---|
| Hypromellose (Opadry® Pink 03F540018) | 4.00 |
| Water, Purified | q.s. |
| ***Coated Tablet weight*** | ***154.00*** |

| | |
|---|---|
| Opadry® Pink 03FS540018: further ingredients are titanium dioxide, talc, red iron oxide and macrogol | |

### Process:

A dry mix containing lactose monohydrate and maize starch was treated with a binder solution containing hypromellose dissolved in water and mixed in a High Shear Mixer Granulator. The wet mass was dried in a Fluid Bed drier and the obtained granules were milled. Thereafter, the granules were blended with solifenacin succinate, hypromellose and magnesium stearate to obtain a blend which was subjected to compaction. The compacted blend was subsequently milled, sifted and then mixed with magnesium stearate to obtain a tablet mass which was compressed into tablets. Finally, the tablets were film-coated.

### Examples 4-6

| Ingredients | Milligram/Tablet | | |
|---|---|---|---|
| | **Ex. 4** | **Ex. 5** | **Ex. 6** |
| **Stage - A (Dry mix)** | | | |
| Lactose Monohydrate (Granulac® 200) | 115.00 | 115.00 | 115.00 |
| Maize Starch | 15.00 | 15.00 | 15.00 |
| **Stage - B (Granulation)** | | | |
| Hypromellose (HPMC E5) | 3.00 | 3.00 | 3.00 |
| Water, Purified | q.s. | q.s. | q.s. |
| **Stage - C (Blending & Compaction)** | 133.00 | 133.00 | 133.00 |
| Excipient Granules | 133.00 | 133.00 | 130.00 |
| Solifenacin Succinate | 10,00 | 10.00 | 10.00 |
| Hypromellose (HPMC E5) | 4.00 | 4.00 | 4.00 |
| Glycerol Dibehenate (Compritol® 888 ATO) | 1.00 | - | 1.00 |
| Glycerol distearate (Type I) (Precirol® ATO 5) | - | 1.00 | - |
| Colloidal Anhydrous Silica (Aerosil® 200) | - | - | 1.00 |
| **Stage - D (Blending & Lubrication)** | | | |
| Stage C compacted blend | 148.00 | 148.00 | 146.00 |
| Silica, Colloidal Anhydrous (Aerosil® 200) | - | - | 2.00 |
| Glycerol Dibehenate (Compritol® 888 ATO) | 2.00 | - | 2.00 |
| Glycerol distearate (Type I) | - | 2.00 | - |
| (Precirol® ATO 5) | | | |
| ***Core Tablet weight*** | 150.00 | 150.00 | 150.00 |
| Stage - E (Coating) | | | |
| Hypromellose Opadry® Pink 03F540018 | 4.00 | 4.00 | 4.00 |
| Water, Purified | q.s. | q.s. | q.s. |
| ***Coated Tablet weight*** | 154.00 | 154.00 | 154.00 |

| | | | |
|---|---|---|---|
| Opadry® Pink 03FS540018: further ingredients are titanium dioxide, talc, red iron oxide and macrogol | | | |

### Process:

A dry mix containing lactose monohydrate and maize starch was treated with a binder solution containing hypromellose dissolved in water and mixed in a High Shear Mixer Granulator. The wet mass was dried in a Fluid Bed drier and the obtained granules were milled. Thereafter, the granules were blended with solifenacin succinate, hypromellose and glycerol dibehenate or glycerol distearate or a mixture of glycerol dibehenate and silicon dioxide to obtain a blend which was subjected to compaction. The compacted blend was subsequently milled, sifted and then mixed with glycerol dibehenate or glycerol distearate or a mixture of glycerol dibehenate and silicon dioxide to obtain a tablet mass which was compressed into tablets. Finally, the tablets were film-coated.

### Examples 7-10

| Ingredients | Milligram/Tablet | | | |
|---|---|---|---|---|
| | **Ex. 7** | **Ex. 8** | **Ex. 9** | **Ex. 10** |
| **Stage - A (Dry Mix)** | | | | |
| Lactose monohydrate (Granulac 200) | 42.50 | 73.50 | 87.50 | 97.50 |
| Maize Starch | 15.00 | 15.00 | 15.00 | 15.00 |
| **Stage** - **B (Granulation)** | - | - | - | |
| Hypromellose (HPMC E5) | 2.00 | 2.00 | 2.00 | 2.00 |
| Water, Purified | q.s | q.s | q.s | q.s |
| **Stage - C (Blending & Lubrication)** | - | - | - | |
| Excipient Granules | 59.50 | 90.50 | 104.50 | 114.50 |
| Solifenacin Succinate | 10.00 | 10.00 | 10.00 | 10.00 |
| Lactose monohydrate | 75.00 | 44.00 | 30.00 | 20.00 |
| Hypromellose (HPMC E5) | 4.00 | 4.00 | 4.00 | 4.00 |
| Magnesium Stearate | 1.50 | 1.50 | 1.50 | 1.50 |
| ***Core Tablet weight*** | 150.00 | 150.00 | 150.00 | 150.00 |
| **Stage** - **D (Coating)** | | | | |
| Hypromellose (Opadry® Pink 03FS540018) | 4.00 | 4.00 | 4.00 | 4.00 |
| Water, Purified | q.s | q.s | q.s | q.s |
| ***Coated Tablet weight*** | 154,00 | 154.00 | 154.00 | 154.00 |

| | | | | |
|---|---|---|---|---|
| Opadry® Pink 03FS540018; further ingredients are titanium dioxide, talc, red iron oxide and macrogol | | | | |

### Process:

A dry mix containing lactose monohydrate and maize starch was treated with a binder solution containing hypromellose dissolved in water and mixed in a High Shear Mixer Granulator. The wet mass was dried in a Fluid Bed drier and the obtained granules were milled. Thereafter, the granules were blended with solifenacin succinate, lactose monohydrate, hypromellose and magnesium stearate to obtain a tablet mass which was compressed into tablets, Finally, the tablets were film-coated.

### Examples 11 and 12

| Ingredients | Milligram/Tablet | |
|---|---|---|
| | **Ex. 11** | **Ex. 12** |
| **Stage** - **A (Dry mix)** | | |
| Lactose Monohydrate (Granular® 200) | 84.80 | 84.00 |
| Maize Starch | 8.00 | 8.00 |

| **Stage - B (Granulation)** | | |
|---|---|---|
| Hypromellose (HPMC E5) | 2.00 | 2.00 |
| Disodium EDTA | 0.20 | - |
| Water, Purified | q.s. | q.s. |

| **Stage - C (Blending & Lubrication)** | | |
|---|---|---|
| Excipient Granules | 95.00 | 94.00 |
| Solifenacin Succinate | 10.00 | 10.00 |
| Lactose Monohydrate (Granulac® 200) | 30.00 | 30.00 |
| Maize Starch | 8.00 | 8.00 |
| Hypromellose (HPMC E5) | 3.00 | 3.00 |
| Magnesium Stearate | 1.00 | 1.00 |
| Colloidal Anhydrous Silica (Aerosil® 200) | 3.00 | 3.00 |
| Propyl Gallate | - | 1.00 |
| ***Core Tablet weight*** | 150.00 | 150.00 |

| **Stage - D (Coating)** | | |
|---|---|---|
| Hypromellose (Opadry® Pink 03F540018) | 4.00 | 4.00 |
| Water, Purified | q.s. | q.s. |
| ***Coated Tablet weight*** | 154.00 | **154.00** |

| | | |
|---|---|---|
| Opadry® Pink 03FS540018: further ingredients are titanium dioxide, talc, red iron oxide and macrogol | | |

### Process:

A dry mix containing lactose monohydrate and maize starch was treated with a binder solution containing hypromellose dissolved in water and mixed in a High Shear Mixer Granulator. The wet mass was dried in a Fluid Bed drier and the obtained granules were milled. Thereafter, the granules were blended with solifenacin succinate, lactose monohydrate, maiz starch, hypromellose, colloidal anhydrous silica and magnesium stearate to obtain a tablet mass which was compressed into tablets. Finally, the tablets were film-coated. The tablets contain disodium EDTA or propyl gallate as antioxidants. While disodium EDTA was contained in the binder solution, and thus constitutes an intragranular component, propyl gallate was added to the tablet mass, and thus constitutes an extragranular component of the tablet.

## Claims

1. Pharmaceutical composition in the form of a solid oral dosage form comprising solifenacin or a pharmaceutically acceptable salt thereof as a pharmaceutically active ingredient and excipient granules, wherein the composition is obtained by a process comprising the method steps of:
i) preparing excipient granules from at least two pharmaceutical excipients by wet granulation,
ii) mixing said excipient granules with the pharmaceutically active ingredient and optionally with additional pharmaceutical excipients to obtain a mixture,
iii) subjecting the mixture obtained in method step (ii) to compression, and
iv) optionally milling the compacted mass obtained in method step (iii), optionally mixing the milled mass with pharmaceutical excipients, and subjecting the milled mass/mixture to compression.

2. Pharmaceutical composition according to claim 1, wherein the pharmaceutically active ingredient is crystalline solifenacin succinate.

3. Pharmaceutical composition according to claim 1 or 2, wherein the excipient granules contain at least one filler, such as microcrystalline cellulose, powdered cellulose, glucose, lactose (anhydrous or monohydrate), maltose, sucrose, isomalt, lactitol, maltitol, mannitol, sorbitol, xylitol, starch (maize, potato, wheat), and calcium hydrogen phosphate, and at least one binder, such as methyl cellulose, hydroxypropylmethyl cellulose, hydroxypropyl cellulose, low-substituted hydroxypropyl cellulose, carboxymethylcellulose sodium, polyethylene glycol, maltodextrin, pregelatinized starch, polyvinylpyrrolidone, vinylpyrrolidone/vinyl acetate copolymer, and polyvinyl alcohol/polyethylene glycol graft copolymer.

4. Pharmaceutical composition according to any one of the preceding claims in the form of an optionally film-coated tablet.

5. Pharmaceutical composition according to claim 4, wherein the tablet or tablet core consists of said excipient granules, said pharmaceutically active ingredient and at least one pharmaceutical excipient selected from lubricants and glidants.

6. Pharmaceutical composition according to any one of the preceding claims, wherein the water content of the composition is 1 % to 10%, preferably 2% to 8%, more preferred 3% to 6% and most preferred 4% to 5% as determined by Karl Fischer titration.

7. A process for preparing a pharmaceutical composition according to any one of the preceding claims, comprising the method steps of:
i) preparing excipient granules from at least two pharmaceutical excipients by wet granulation,
ii) mixing said excipient granules with the pharmaceutically active ingredient and optionally with additional pharmaceutical excipients to obtain a mixture,
iii) subjecting the mixture obtained in method step (ii) to compression, and
iv) optionally milling the compacted mass obtained in method step (iii), optionally mixing the milled mass with pharmaceutical excipients, and subjecting the milled mass/mixture to compression.

## Patentansprüche

1. Pharmazeutische Zusammensetzung in Form einer festen oral zu verabreichenden Darreichungsform, umfassend Solifenacin oder ein pharmazeutisch verträgliches Salz davon als pharmazeutischen Wirkstoff und ein Hilfsstoffgranulat, wobei die Zusammensetzung durch ein Verfahren erhalten wird, welches die folgenden Verfahrensschritte umfasst:
i) Herstellen eines Hilfsstoffgranulats aus mindestens zwei pharmazeutischen Hilfsstoffen durch Feuchtgranulierung,
ii) Mischen des Hilfsstoffgranulats mit dem pharmazeutischen Wirkstoff und gegebenenfalls mit zusätzlichen pharmazeutischen Hilfsstoffen, um eine Mischung zu erhalten,
iii) Verpressen der im Verfahrensschritt (ii) erhaltenen Mischung, und
iv) gegebenenfalls Mahlen der im Verfahrensschritt (iii) erhaltenen kompaktierten Masse, gegebenenfalls Mischen der gemahlenen Masse mit pharmazeutischen Hilfsstoffen, und Verpressen der gemahlenen Masse/Mischung.

2. Pharmazeutische Zusammensetzung nach Anspruch 1, wobei der pharmazeutische Wirkstoff kristallines Solifenacinsuccinat ist.

3. Pharmazeutische Zusammensetzung nach Anspruch 1 oder 2, wobei das Hilfsstoffgranulat mindestens einen Füllstoff, wie mikrokristalline Cellulose, pulverförmige Cellulose, Glucose, Lactose (wasserfrei oder Monohydrat), Maltose, Saccharose, Isomalt, Lactitol, Maltitol, Mannitol, Sorbitol, Xylitol, Stärke (Mais, Kartoffel, Weizen) und Calciumhydrogenphosphat, und mindestens ein Bindemittel, wie Methylcellulose, Hydroxypropylmethylcellulose, Hydroxypropylcellulose, niedrig substituierte Hydroxypropylcellulose, Carboxymethylcellulose-Natrium, Polyethylenglycol, Maltodextrin, vorverkleisterte Stärke, Polyvinylpyrrolidon, Vinylpyrrolidon/Vinylacetat-Copolymer und Polyvinylalkohol/Polyethylenglycol-Pfropfcopolymer, enthält.

4. Pharmazeutische Zusammensetzung nach einem der vorhergehenden Ansprüche in Form einer gegebenenfalls filmbeschichteten Tablette.

5. Pharmazeutische Zusammensetzung nach Anspruch 4, wobei die Tablette oder der Tablettenkern aus dem Hilfsstoffgranulat, dem pharmazeutischen Wirkstoff und mindestens einem Hilfsstoff, ausgewählt aus Schmiermitteln und Gleitmitteln, besteht.

6. Pharmazeutische Zusammensetzung nach einem der vorhergehenden Ansprüche, wobei der Wassergehalt der Zusammensetzung, mittels Karl-Fischer-Titration bestimmt, 1% bis 10%, vorzugsweise 2% bis 8%, stärker bevorzugt 3% bis 6% und am meisten bevorzugt 4% bis 5% beträgt.

7. Verfahren zur Herstellung einer pharmazeutischen Zusammensetzung nach einem der vorhergehenden Ansprüche umfassend die folgenden Verfahrensschritte:
i) Herstellen eines Hilfsstoffgranulats aus mindestens zwei pharmazeutischen Hilfsstoffen durch Feuchtgranulation,
ii) Mischen des Hilfsstoffgranulats mit dem pharmazeutischen Wirkstoff und gegebenenfalls mit zusätzlichen pharmazeutischen Hilfsstoffen, um eine Mischung zu erhalten,
iii) Verpressen der im Verfahrensschritt (ii) erhaltenen Mischung, und
iv) gegebenenfalls Mahlen der im Verfahrensschritt (iii) erhaltenen kompaktierten Masse, gegebenenfalls Mischen der gemahlenen Masse mit pharmazeutischen Hilfsstoffen, und Verpressen der gemahlenen Masse / Mischung.

## Revendications

1. Composition pharmaceutique sous la forme d'une forme posologique orale solide comprenant de la solifénacine ou un de ses sels pharmaceutiquement acceptables comme principe actif et des granules d'excipient, dans laquelle la composition est obtenue par un procédé comprenant les étapes de procédé suivantes :
i) préparer des granules d'excipient à partir d'au moins deux excipients pharmaceutiques par granulation humide,
ii) mélanger lesdits granules d'excipient avec le principe actif et éventuellement avec des excipients pharmaceutiques supplémentaires pour obtenir un mélange,
iii) soumettre le mélange obtenu en l'étape de procédé (ii) à la compression, et
iv) éventuellement, le broyage de la masse compactée obtenue en l'étape de procédé (iii), mélanger facultativement de la masse broyée avec des excipients pharmaceutiques et la compression du mélange / de la masse broyée.

2. Composition pharmaceutique selon la revendication 1, dans laquelle le principe actif est le succinate de solifénacine cristallin.

3. Composition pharmaceutique selon la revendication 1 ou 2, dans laquelle les granules d'excipient contiennent au moins un agent diluant, telle que la cellulose microcristalline, la cellulose en poudre, le glucose, le lactose (anhydre ou monohydraté), le maltose, le saccharose, l'isomalt, le lactitol, le maltitol, le mannitol, le sorbitol, le xylitol, l'amidon (maïs, pomme de terre, blé), et l'hydrogénophosphate de calcium, et au moins un agent liant, tel que la méthylcellulose, l'hydroxypropylméthylcellulose, l'hydroxylpropylcellulose, l'hydroxypropylcellulose faiblement substituée, carboxyméthylcellulose sodique, polyéthylène glycol, maltodextrine, amidon prégélatinisé, polyvinylpyrrolidone, vinylpyrrolidone/vinylacétate copolymère, et le copolymère greffé alcool polyvinylique/polyéthylène glycol.

4. Composition pharmaceutique selon l'une quelconque des revendications précédentes, sous la forme d'un comprimé éventuellement pelliculé.

5. Composition pharmaceutique selon la revendication 4, dans laquelle le comprimé ou le noyau de comprimé est constitué desdits granules d'excipient, dudit principe actif et d'au moins un excipient pharmaceutique choisi parmi les agents lubrifiants et les agents glissants.

6. Composition pharmaceutique selon l'une quelconque des revendications précédentes, dans laquelle la teneur en eau de la composition est de 1% à 10%, de préférence de 2% à 8%, mieux encore de 3% à 6% et de manière préférée entre toutes de 4% à 5%, telle que déterminée par Karl Fischer titrage.

7. Procédé de préparation d'une composition pharmaceutique selon l'une quelconque des revendications précédentes, comprenant les étapes de procédé consistant à:
i) préparer des granules d'excipient à partir d'au moins deux excipients pharmaceutiques par granulation humide,
ii) mélanger lesdits granules d'excipient avec le principe actif et éventuellement avec des excipients pharmaceutiques supplémentaires pour obtenir un mélange,
iii) soumettre le mélange obtenu en l'étape de procédé (ii) à la compression, et
iv) éventuellement, le broyage de la masse compactée obtenue en l'étape de procédé (iii), mélanger facultativement de la masse broyée avec des excipients pharmaceutiques et la compression du mélange / de la masse broyée.
